# EUROPEAN PATENT SPECIFICATION

(11) **EP 3 808 316 B1**
(45) Date of publication and mention of the grant of the patent: **29.01.2025**
(21) Application number: 18922575.8
(22) Date of filing: 09.08.2018
(51) Int. Cl.: A61F 2/95, A61B 18/14, A61L 29/02, A61L 29/14, A61B 17/00, A61L 15/00, A61L 29/00

(54) **HOT-PUNCTURE STENT IMPLANTATION DEVICE**
VORRICHTUNG ZUR STENTIMPLANTATION DURCH HEISSPUNKTION
DISPOSITIF D'IMPLANTATION D'ENDOPROTHÈSE À PERFORATION À CHAUD

(30) Priority: 13.06.2018 CN 201810606565
(43) Date of publication of application: 21.04.2021
(73) Proprietor: Micro-Tech (Nanjing) Co., Ltd., Nanjing, Jiangsu 210032 (CN); Shengjing Hospital of China Medical University, Shenyang, Liaoning 110004 (CN)
(72) Inventor: SUN, Siyu, Nanjing, Jiangsu 210032 (CN); GE, Nan, Nanjing, Jiangsu 210032 (CN); GUO, Jintao, Nanjing, Jiangsu 210032 (CN); WEI, Jianyu, Nanjing, Jiangsu 210032 (CN); SHEN, Zhenghua, Nanjing, Jiangsu 210032 (CN); LI, Changqing, Nanjing, Jiangsu 210032 (CN); LENG, Derong, Nanjing, Jiangsu 210032 (CN); SUN, Jialing, Nanjing, Jiangsu 210032 (CN); LIU, Chunjun, Nanjing, Jiangsu 210032 (CN)
(74) Representative: Jacob, Reuben Ellis
(86) International application number: PCT/CN2018/099489
(87) International publication number: WO 2019/237481

(56) References cited:
- WO-A1-2017/142236
- WO-A1-2018/093114
- WO-A1-2018/093114
- CN-A- 101 605 509
- CN-A- 103 142 336
- CN-A- 104 519 837
- CN-A- 105 658 182
- CN-U- 203 122 693
- FRANK WEILERT ET AL: "Specially designed stents for translumenal drainage", GASTROINTESTINAL INTERVENTION, vol. 4, no. 1, 1 June 2015 (2015-06-01), pages 40 - 45, XP055554091, ISSN: 2213-1795, DOI: 10.1016/j.gii.2015.03.003

## Description

### TECHNICAL FIELD

The present invention relates to a thermopuncture implantation device in the field of medical apparatuses, and in particular, to a thermopuncture stent implantation device, which integrates cutting and injection functions.

### BACKGROUND

Digestive tract-gallbladder anastomosis is that under an endoscope, a thermal implantation device punctures into the gallbladder at a target location through a gastric wall or a duodenal wall, a distal end of a fully covered double mushroom head stent is placed in the gallbladder, and a proximal end of mushroom heads is placed in the stomach or duodenum, so as to open up a passage between the digestive tract and the gallbladder, in other words, to recreate a new path between the digestive tract and the gallbladder. Afterwards, through a gastroscope and the newly built passage, stones in the gallbladder are removed by a stone removal basket, so as to achieve endoscopic gallbladder preservation and stone removal surgery, which provides a new treatment problem for patients with a gallbladder disease who are not suitable for surgery, and can also provide patients having good gallbladder function with a treatment method that can preserve the gallbladder function, improving the long-term quality of life of the patients. The stomach-pancreatic pseudocyst stent anastomosis is that under an endoscope, a large-diameter fully covered double mushroom head stent punctures into a pancreatic pseudocyst of a patient through the stomach, and is placed therein, so as to achieve the anastomosis between the stomach and the pancreatic pseudocyst, thereby fully draining the hydrops and sphacelus in the pancreatic pseudocyst.

In the Duodenum-bile duct anastomosis, a traditional ERCP surgery is inserting a guide wire or other instrument into the duodenal papilla retrogradely from the duodenum through an ERCP endoscopy, to reach the common bile duct, and performing stones removal and biopsy treatment on the common bile duct, etc. For a patient into whose body the guide wire is difficult to insert, percutaneous puncture or surgical operation is usually required, which may lower the patient's quality of life or bring a greater trauma.

Regarding gastrointestinal anastomosis, a patient who has been vomiting because a passage of food in the stomach into the intestine is blocked due to tumor invasion, is either subjected to laparotomy to establish a new gastrointestinal passage, or can only rely on intravenous nutrition for support, in the past. For those patients who are old or whose physical conditions are no longer suitable for laparotomy, their quality of life is extremely low, which also bring a heavy burden to their families. Gastrointestinal anastomosis is that under an endoscopic ultrasonography scope (EUS), a large diameter fully covered double mushroom head stent punctures into a nearness small bowel through the stomach, and is placed therein, to open up a passage between the stomach and the small intestine, in other words, to recreate a new path between the stomach and the small intestine, thereby solving influence of duodenal obstruction on the life of patients.

In the past, such "bypass" construction requires laparotomy under general anesthesia, which is more traumatic. A minimally invasive surgery under an endoscopy has less trauma, short operation time, small pain and quick recovery, which fully shows the advantages of endoscopic minimally invasive surgery. In recent years, with the continuous development and upgrading of endoscopic technology and various instrument accessories, the endoscopy plays an increasingly important role in the diagnosis and treatment of various diseases of the digestive system, especially the continuous innovation of the minimally invasive surgery under endoscopy provides a new minimally invasive treatment method for many patients with gastrointestinal and biliary and pancreatic diseases who are unable or unwilling to undergo a surgery. Currently, in the above four traditional surgeries, the stent is usually a metal double mushroom head stent with a diameter of ϕ10mm-ϕ16mm, and an outer diameter of a matching thermal implantation device is ϕ3.5mm-ϕ3.6mm (10.5Fr-10.8Fr), and a traditional ultrasound endoscopic channel is ϕ3.7mm, because the gap is too small, a traditional charged implantation device cannot move freely back and forth in the endoscopic channel, which is a main reason why the above surgeries are difficult to perform. At the same time, the outer diameter of an ultrasound endoscope is ϕ14mm, which is 4mm larger than the outer diameter of a traditional gastroscope (ϕ10mm), thus it is more inconvenient to operate and has relatively fewer places to reach.

Therefore, in order to carry out a stomach gallbladder anastomosis, a gastrointestinal anastomosis, and a human body natural orifice transluminalendoscopic surgery (NOTES), etc., through gastroscope, it is necessary to design a smaller charged implantation device, and simplify release step of the stent through a gastroscopic channel, so as to release the stent more safely and quickly.

WO2018/093114A1 discloses a stent delivery system comprising: a connector portion connected to an external current source; an electrocautery tip connected to the connector portion by a wire; and a delivery portion, one side of which is linked with the electrocautery tip, the other side of which is linked with the connector portion, and in which the wire is arranged to connect the electrocautery tip and the connector portion, wherein a stent space portion is formed adjacent to the electrocautery tip inside the delivery portion such that a stent is arranged therein.

CN203122396U discloses an integral stent implantation device, comprising a front handle and a rear handle, a front end of the front hand being provided with an outer tube which is freely connected with a cutting head at its top end, a middle tube and a support being provided inside the outer tube; and also discloses a stainless steel tube.

Frank Weilert and Kenneth F. Binmoeller ("Specially designed stents for translumenal drainage", GASTROINTESTINAL INTERVENTION, vol 4, no.1, 1 June 2015, pages 40-45) discloses that endoscopic ultrasonography (EUS)-guided translumenal drainage of pancreatic fluid collections and obstructed bile and pancreatic ducts has been widely practiced for over a decade now, using conventional tubular plastic and metal stents. Their application for transmural drainage has been "off label" and limited by the lack of lumen-to-lumen anchorage that can lead to leakage, perforation, and stent migration. In addition, the length of a tubular stent exceeding the anatomical requirement of a translumenal anastomosis can lead to tissue trauma at the stent ends.

### SUMMARY

The present invention is defined in the appended set of claims. The present invention provides a brand-new method to solve bile duct obstruction, and meanwhile the method saves surgery time, saves surgical instruments, reduces the difficulty of surgery, providing possibility for more doctors to carry out this surgery. A thermopuncture stent implantation device according to the present invention eliminates an inner tube and a conductive wire of a traditional implantation device, and replaces them with a conductive part, which achieves the purpose of supporting the stent and meanwhile has the function of transmitting high-frequency electricity. An outer diameter of the existing thermopuncture implantation device can be reduced from 3.5mm-3.6mm (10.5Fr-10.8Fr) to 3.15mm (9.5Fr), so that the thermopuncture implantation device can pass through a traditional gastroscopic channel of ϕ3.2mm, providing possibility for doctors to perform more advanced digestive tract-gallbladder anastomosis, duodenum-bile duct anastomosis, stomach-pancreatic pseudocyst stent anastomosis, gastrointestinal anastomosis, NOTES surgery and so on.

In the following, one end of a conductive head is defined as a distal end, and an end of the implantation device connected to an external power source is defined as a proximal end.

The thermopuncture stent implantation device has a proximal end and a distal end, a distal end of a front handle is provided with an outer tube, the outer tube extends from the proximal end to the distal end, an outer diameter of the distal end of an outer tube is less than or equal to 3.15 mm, an insulating middle tube is provided in the outer tube, and extends from the proximal end to the distal end, a conductive part is provided in the insulating middle tube, the insulating middle tube and the conductive part extend from the proximal end to the distal end, a terminal of the proximal end of the conductive part can be connected to an external power source; a boosting tube is provided between the proximal end of the outer tube and the insulating middle tube, the distal end of the boosting tube and the proximal end of the insulating middle tube are connected with each other; the distal end of the conductive part is provided with an insulating part, a conductive head is distributed on the insulating part, and the conductive head is connected with the conductive part to achieve a conductive function, and the stent, after being compressed, is located in a space between the distal end of the conductive part and the outer tube, and the front handle is connected to the proximal end of the outer tube, and moved backwards along the boosting tube, to drive the outer tube to move backwards to release the stent. The conductive part not only conducts electricity, but also supports the stent. Compared with a traditional stent implantation device, the conductive part reduces an inner tube and a guide wire, and at the same time, it can conduct electricity, cut tissues, and release the stent after reaching a lesion site.

There is a certain gap between the insulating part and the conductive part, the conductive head is provided at a terminal of the distal end of the implantation device, one end of the conductive head can extend from the distal end to the proximal end to enter the gap between the insulating part and the conductive part, and thus be connected with the conductive part to achieve a conductive function, the other end of the conductive head is covered on an outer surface of the insulating part.

Preferably, the conductive part is a hollow conductive part.

More preferably, the terminal of the proximal end of the conductive part is connected with a Luer connector to achieve liquid injection.

Preferably, the conductive part is a conductive wire.

Preferably, the conductive part is a nickel-titanium wire.

Preferably, the conductive part is a metal material. More preferably, the conductive part is a stainless steel material.

Preferably, the material of the insulating part is ceramic.

The outer tube includes a proximal outer tube and a distal outer tube, the proximal outer tube and the distal outer tube are connected in a taper. The boosting tube extends towards the proximal end and is connected with a rear handle, and a positioning part is provided between the front handle and the rear handle. An outer surface of the conductive part at a certain distance from the conductive head is covered with a resistance part. The conductive head comprises two or four conductive wires, and the two or four conductive wires are evenly distributed within a groove on an outer surface of the insulating part. The other end of the conductive head close to an outer side is completely covered on the outer surface of the insulating part, and when cutting with the conductive head, a cut surface of a wound is a circular surface. An outer surface of the conductive part can be covered with a riveting tube, an end of the conductive head can extend from the distal end to the proximal end to enter the gap between the insulating part and the conductive part, and achieve the conductive function by connection of the riveting tube and the conductive part.

### Beneficial effects:

The outer diameter of the thermopuncture stent according to the present invention is smaller than the outer diameter of the stent implantation device in the prior art, and provides a new minimally invasive treatment method for many patients with gastrointestinal and biliary and pancreatic diseases who are unable or unwilling to undergo a surgery.

The thermopuncture implantation device (diameter of 3.15 mm) according to the present invention can accommodate a double mushroom head metal stent that is braided by a nickel-titanium wire and has a diameter of ϕ10mm-ϕ16mm, and can enter into stomach, duodenum and other organs through a traditional gastroscopic channel of 3.2mm; the implantation device is electrified to puncture a stomach wall or an intestinal wall, and enter into the small intestine, gallbladder, pancreatic cyst, common bile duct and other structures, to release the stent precisely, and it can anastomose the above tissues with the stomach wall or the intestinal wall respectively, to achieve drainage, gallbladder protection, stone removal, bypass opening and other functions.

It can be inferred from the above that in the case where the traditional ultrasound endoscopic channel is ϕ3.7mm, when the outer diameter of the implantation device of the present disclosure is increased from 3.15mm (9.5Fr) to 3.5mm-3.6mm (10.5Fr-10.8Fr), then a cross-sectional area of the implantation device will be increased by 23-31%, as calculated by the formula (π*R1*R1)/(π*R2*R2), where R1=3.5/2 or 3.6/2, and R2=3.15/2, so that a double mushroom head metal stent that is braided by a nickel-titanium wire and has a larger diameter (e.g., ϕ18mm) than diameter ϕ16mm can be fitted into the thermopuncture implantation device of the present disclosure. For example, When the diameter of the stent is 18mm, (π*R3*R3)/(π*R4*R4)=126% where R3=18/2mm, R4=16/2mm, that is, a cross-sectional area of the stent with a diameter of 18mm is increased by 26% compared with the stent with a diameter of 16mm. Since the increase of the cross-sectional area of the implantation device is 23-31%, the stent with a cross-sectional area increase of 26% can be placed into the implantation device.

### BRIEF DESCRIPTION OF THE DRAWINGS

FIG. 1A is a cross-sectional schematic diagram of an implantation device;
FIG. 1B is a schematic structural diagram of a distal end of a cross-section of an implantation device;
FIG. 2 is an overall schematic diagram of an implantation device product;
FIG. 3A is a cross-sectional schematic diagram taken along B-B in FIG. 1B when a conductive part is a hollow conductive part;
FIG. 3B is a cross-sectional schematic diagram taken along C-C in FIG. 1B when a conductive part is a hollow conductive part;
FIG. 4A is a cross-sectional schematic diagram taken along B-B in FIG. 1B when a conductive part is a conductive wire;
FIG. 4B is a cross-sectional schematic diagram taken along C-C in FIG. 1B when a conductive part is a conductive wire;
FIG. 5A is a cross-sectional diagram of a proximal tail structure of a stent implantation device corresponding to FIG. 3A and FIG. 3B;
FIG. 5B is a partial enlarged diagram of FIG. 5A;
FIG. 6 is a structural cross-sectional diagram of a proximal tail of a stent implantation device corresponding to FIG. 4A and FIG. 4B;
FIGS. 7A-7D are schematic diagrams of distal ends of different types of implantation devices;
FIGS. 8A-8B are a schematic diagram of a distal end of an integrated implantation device;
FIGS. 9A-9B are a schematic diagram of a distal end of a split implantation device;
FIGS. 10A-10C are a schematic diagram of a distal end of a flanged implantation device;
FIG. 11 is a schematic diagram of a safety buckle; and
FIG. 12 is a schematic diagram of a double mushroom head stent fully opened.

11-conductive head, 12-insulating part, 13-conductive part, 21-outer tube, 211-proximal outer tube, 212-distal outer tube, 22-boosting tube, 23-insulating middle tube, 24-safety buckle, 25-outer tube locking cap, 26-safety lock, 27-positioning part, 28-resistance part, 29-riveting tube, 30-front handle, 31-rear handle, 32-conductive base, 33-conductive plug, 34-Luer connector, 40-distal tissue, 41-proximal tissue, and 42-double mushroom head stent.

### DESCRIPTION OF EMBODIMENTS

In order to make the purpose, technical solutions and advantages of the present invention more explicit, the present invention will be further illustrated in detail in combination with accompanying drawings and embodiments hereinafter. It should be understood that specific embodiments described herein are only used for explaining the present invention, instead of limiting the present invention.

In the following, an end of a conductive head is defined as a distal end, and an end of a stent implantation device connected to an external power source is defined as a proximal end.

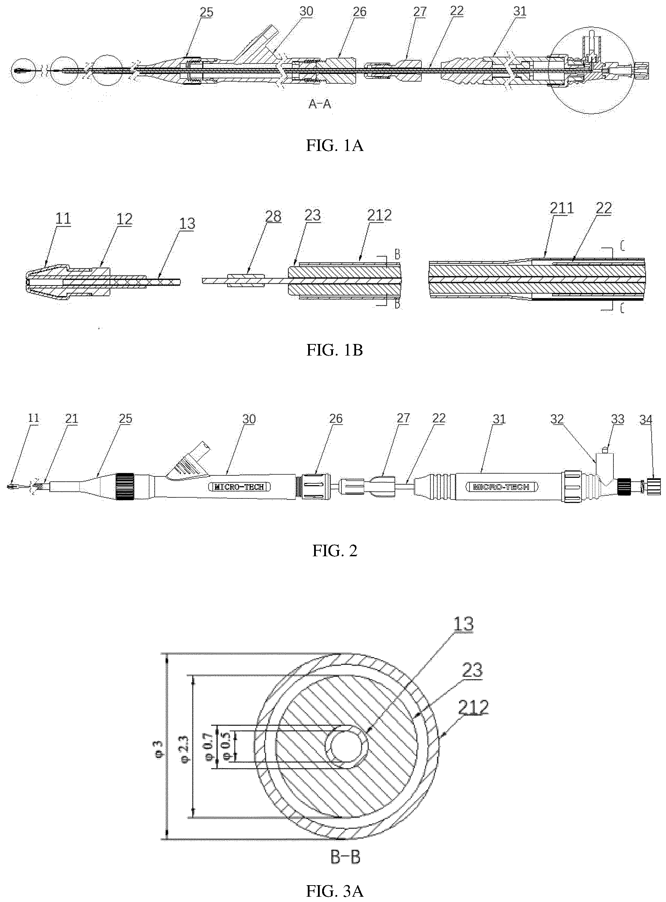

As shown in FIG. 1A, FIG. 1B and FIG. 2, the stent implantation device according to the present invention has the proximal end and the distal end, and the stent implantation device includes an outer tube 21, a boosting tube 22, an insulating middle tube 23, an outer tube locking cap 25, a safety lock 26, a positioning part 27, a resistance part 28, a front handle 30, a rear handle 31, a conductive base 32, a conductive plug 33, a Luer connector 34, a conductive head 11, an insulating part 12 and a conductive part 13.

The outer tube 21 includes a proximal outer tube 211 and a distal outer tube 212. The proximal outer tube 211 is provided at the distal end of the front handle 30, and can be fixed with the front handle 30 through the outer tube locking cap 25, the safety lock 26 is provided at the proximal end of the front handle 30, and the safety lock 26 has threads, which can be matched with threads on the proximal end of the front handle 30, and installed thereon. The insulating middle tube 23 and a stent are arranged within the outer tube 21, the proximal end of the stent abuts against the distal end of the insulating middle tube 23, and the distal end of the stent is close to the insulating part 12, leaving a certain gap; the proximal outer tube 211 and the distal outer tube 212 are connected in a taper. The boosting tube 22 is provided between the proximal outer tube 211 and the insulating middle tube 23, the boosting tube 22 can be made of a stainless steel material, the distal end of the boosting tube 22 and the proximal end of the insulating middle tube 23 are connected with each other; such taper design of the proximal outer tube 211 and the distal outer tube 212 makes the size of the distal outer tube 212 entering a lesion site less than or equal to 3.15 mm, and the boosting tube 22 is provided between the proximal outer tube 211 and the insulating middle tube 23, to provide a force required to release the stent. The insulating middle tube 23 can be made from a special polymer material polyether ether ketone, has high-performance electrical insulating property and thus can isolate the high-frequency electricity of the conductive part 13 from the boosting tube 22, so that the operator can completely avoid the risk of electric shock. The boosting tube 22 extends towards the proximal end and is connected with the rear handle 31, the conductive base 32 is provided at the proximal end of the rear handle 31, there is the conductive plug 33 within the conductive base 32, and the conductive plug 33 can be connected to the conductive head 11 through the conductive part 13, so as to achieve electrifying.

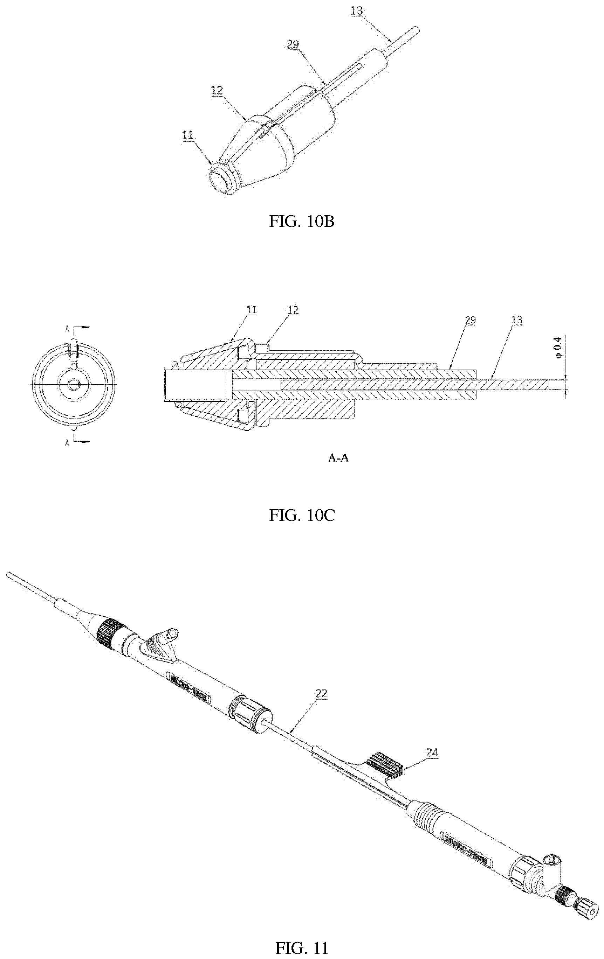

The positioning part 27 may be further provided between the front handle 30 and the rear handle 31, the positioning part 27 can be designed as a structure of a safety buckle 24. As shown in FIG. 11A and FIG. 11B, the positioning part 27 is the structure of the safety buckle 24, and when releasing the stent, the safety lock 26 is first loosed to move backwards the front handle 30 towards the proximal end, so as to touch the safety buckle 24, the distal end of the stent is released within the distal tissue 40, the stent implantation device is withdrawn, to pull the stent to close to the proximal tissue, and remove the safety buckle 24. The front handle 30 is continued to be withdrawn towards the proximal end, and the stent is continued to be released in the proximal tissue 41, so as to achieve an anastomosing connection of the distal tissue 40 with the proximal tissue 41 by the stent.

An outer surface of the conductive part 13 at a certain distance from the conductive head 11 can be covered with the resistance part 28. The resistance part 28 can provide a certain resistance for the stent when the stent is released, so that the stent is not easy to slip to the outside of the lesion.

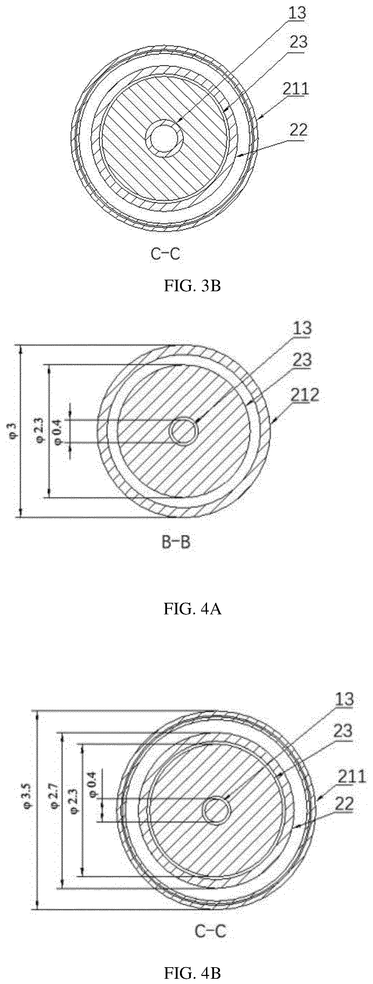

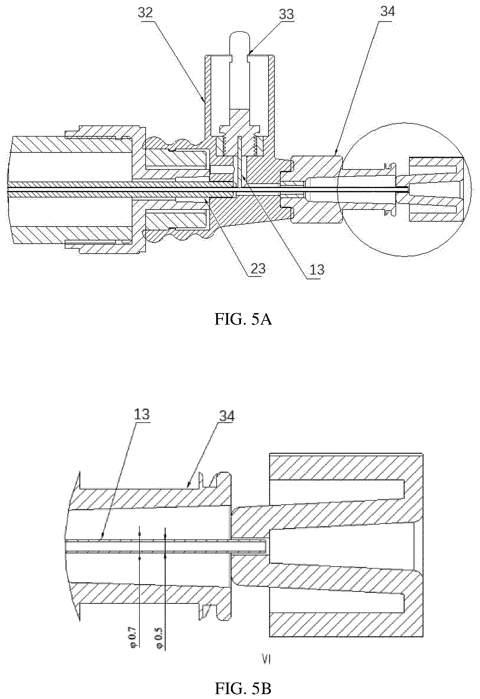

The distal end of the stent implantation device further includes the conductive head 11, the insulating part 12 and the conductive part 13. When the conductive plug 33 is connected to an external high-frequency power source, the high-frequency power source is transmitted to the conductive head 11 through the conductive part 13, so that the stent implantation device has electrical cutting function, to perform a high-frequency cutting on a human tissue. The conductive part 13 can be any kind of medical metal material, such as nickel titanium material or stainless steel material; the conductive part 13 is arranged within the insulating middle tube 23, extends from the distal end to the proximal end, and is connected to the conductive plug 33 through the rear handle 31, the size of the outer diameter of the conductive part 13 can be designed according to actual needs, the present invention can reduce an outer diameter of an implantation part of an existing thermopuncture stent implantation device from 3.5mm-3.6mm (10.5Fr-10.8Fr) to below 3.2mm (9Fr) through a design of the conductive part 13, and preferably, it can be reduced to 3.15mm (9.5Fr). In addition, the conductive part 13 can be a hollow conductive part, so as to achieve the function of liquid injection and development, and the conductive part 13 can also be designed as a conductive wire. When the conductive part 13 is designed as a hollow conductive part, a cross-sectional diagram taken along B-B position in FIG. 1B is shown in FIG. 3A, showing a position relation of the conductive part 13, the insulating middle tube 23 and the distal outer tube 212, and a cross-sectional diagram taken along C-C position in FIG. 1B is shown in FIG. 3B, showing a position relation of the conductive part 13, the insulating middle tube 23 and the proximal outer tube 212. FIG. 5A is a cross-sectional view of a proximal tail structure of a stent implantation device corresponding to FIG. 3A and FIG. 3B, FIG. 5B is a partial enlarged view of FIG. 5A, there is the conductive plug 33 within the conductive base 32, and the conductive plug 33 can be connected to the conductive head 11 through the conductive part 13, so as to achieve electrifying. The proximal end of the conductive part 13 communicates with the Luer connector 34, a doctor can connect the Luer connector 34 with a standard injector, and can inject a liquid or a contrast agent into a hollow tube cavity, the liquid or the contrast agent passes through the tube cavity of the conductive part 13 to reach the conductive head 11 at the distal end of the implantation device, and then is injected into a patient's lesion site, the contrast agent is developed under X-ray, marking a target location of the lesion for the doctor, and the doctor can prepare for the next step of releasing the stent.

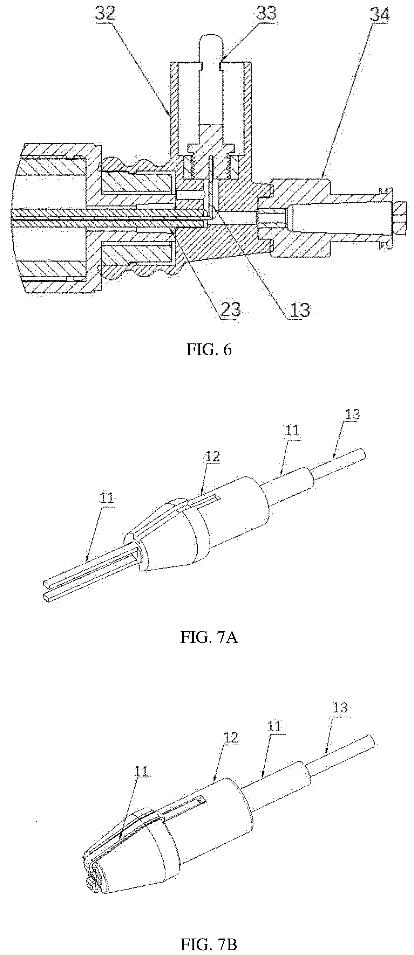

When the conductive part 13 is designed as a conductive wire, the conductive wire can adopt different sizes according to requirements. A cross-sectional diagram taken along B-B position in FIG. 1B is shown in FIG. 4A, showing a position relation of the conductive part 13, the insulating middle tube 23 and the distal outer tube 212; a cross-sectional diagram taken along C-C position in FIG. 1B is shown in FIG. 4B, showing a positional relation of the conductive part 13, the insulating middle tube 23 and the proximal outer tube 211. FIG. 6 is a structural cross-sectional view of a proximal tail of a stent implantation device corresponding to FIG. 4A and FIG. 4B, there is the conductive plug 33 within the conductive base 32, and the conductive plug 33 can be connected to the conductive head 11 through the conductive part 13, so as to achieve electrifying.

The insulating part 12 is located at the distal end of the conductive part 13, there is a certain gap between the insulating part 12 and the conductive part 13, one end of the conductive head 11 can extend from the distal end to the proximal end, to enter the gap between the insulating part 12 and the conductive part 13, so as to be connected with the conductive part 13 to achieve a conductive function, and the other end of the conductive head 11 is covered on an outer surface of the insulating part 12. High-frequency electricity is transmitted to the conductive head 11 at the distal end of the stent implantation device through the conductive part 13, so that the stent implantation device has an electrical cutting function, and can perform a high-frequency cutting and puncture on a human tissue. The insulating part 12 can be made of, such as, a ceramic material, which can prevent tissues from sticking, and make cutting more convenient.

The conductive part 13 according to the present invention replaces an inner tube and a conductive wire of a traditional stent implantation device, having a conductive function, and replacing an outer diameter ϕ1.1mm of an original inner tube and an outer diameter ϕ0.3mm of the original conductive wire with a diameter less than ϕ0.4mm of the conductive part 13, with the total diameter being reduced by a space of ϕ1mm (a space of 3Fr), so that a conventional covered gastrointestinal stent (10mm-16mm) can be installed; and since ϕ3.5mm-ϕ3.6mm (10.5Fr-10.8Fr) of the outer diameter of an original traditional thermal implantation device is reduced to 3.15mm (9.5Fr), an electric implantation device can smoothly pass through a gastroscopic channel of ϕ3.2mm.

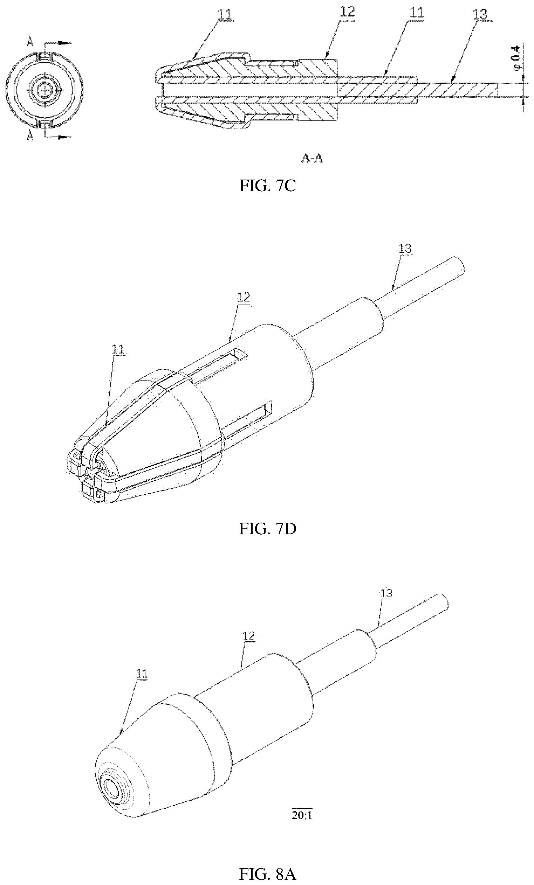

The structures of the conductive head 11, the insulating part 12 and the conductive part 13 at the distal end of the stent implantation device according to the present invention are as shown in FIGS. 7A-7D, the conductive head 11 can comprises two or four conductive wires, one end of the conductive head 11 can extend from the distal end to the proximal end, to enter the gap between the insulating part 12 and the conductive part 13, and thus be connected with the conductive part 13 to achieve a conductive function; the other end of the conductive head 11 is covered on the outer surface of the insulating part 12. At the distal end, the conductive head 11 can be evenly distributed within grooves on an outer surface of the insulating part 12 by the two or four conductive wires, so as to achieve conductive and cutting functions. Within grooves on the outer surface of the insulating part 12, adjacent conductive wires are spaced apart in the same angle, and radially distributed on the outer surface of the insulating part 12.

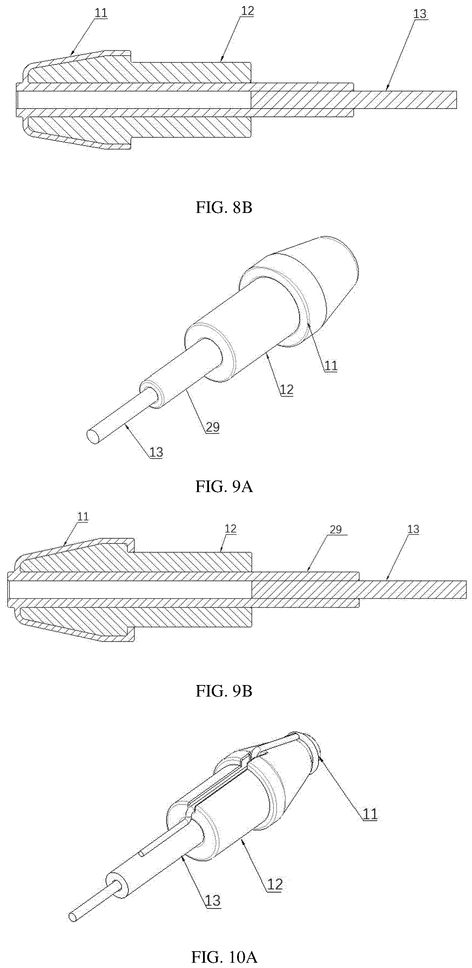

As shown in FIGS. 8A-8B, one end of the conductive head 11 can extend from the distal end to the proximal end, to enter the gap between the insulating part 12 and the conductive part 13, and thus be connected with the conductive part 13 to achieve a conductive function; the other end of the conductive head 11 is fully covered on the outer surface of the insulating part 12. In this case, when cutting with the conductive head 11, a cut surface is a circular surface, instead of a straight incision, thus it is easier to stop bleeding when using a hemostatic clip to stop bleeding, which is beneficial to wound healing.

As shown in FIGS. 9A-9B, the outer surface of the conductive part 13 can be covered with a riveting tube 29, one end of the conductive head 11 can extend from the distal end to the proximal end to enter the gap between the insulating part 12 and the conductive part 13, and can be connected with the conductive part 13 through the riveting tube 29 to achieve a conductive function. The riveting tube 29 can be made of stainless steel, and can connect the conductive part 13 with the insulating part 12. As shown in FIG. 9A, the other end of the conductive head 11 can be fully covered on the outer surface of the insulating part 12, and in this case, when cutting with the conductive head 11, the cut surface is a circular surface, instead of a straight incision, which is beneficial to wound healing.

As shown in FIGS. 10A-10C, the other end of the conductive head 11 can also be distributed within a groove on the surface of the insulating part 12 in the form of one conductive wire, and is looped around a terminal of the distal end of the stent implantation device to form a " 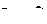 " bevel conductive incision, and at this time, when cutting a tissue, the conductive wire looped and the conductive wire distributed within the groove are utilized. If there is no riveting tube 29, one end of the conductive head 11 is directly connected to the conductive part 13, and the other end of the conductive head 11 is distributed on the periphery of the insulating part 12, which can also achieve the conductive function.

When the stent implantation device according to the present invention is used, after the conductive plug 33 is connected to an external high-frequency power source, the high-frequency power source is transmitted to the conductive head 11 through the conductive part 13, so that the stent implantation device has an electrical cutting function, and can cut the diseased distal tissue 40; if the conductive part 13 is a hollow conductive part, it is connected with an external Luer connector, so as to make the stent implantation device have a liquid injection function.

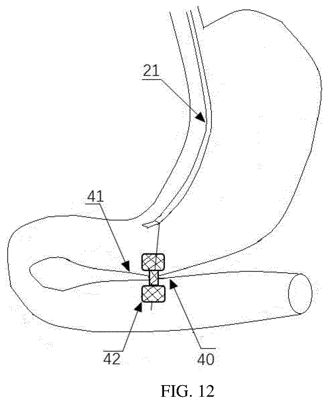

As shown in FIG. 12, the double mushroom head stent 42 is released by the thermopuncture stent implantation device, and when the double mushroom head stent 42 is opened, one end of which is in the distal tissue 40 and the other end of which is in the proximal tissue 41.

The above descriptions are only preferred embodiments of the present application, so that those skilled in the art can understand or implement the invention of the present application. Multiple amendments to these embodiments and combinations thereof will be obvious to those skilled in the art, and general principles defined herein can be achieved in the other embodiments without departing from the scope of the present application,

## Claims

1. An thermopuncture stent implantation device, wherein the thermopuncture stent implantation device has a proximal end and a distal end, a front handle (30), a rear handle (31), the distal end of the front handle (30) is provided with an outer tube (21), the outer tube (21) extends from the proximal end to the distal end, an outer diameter of the distal end of the outer tube (21) is less than or equal to 3.15 mm, an insulating middle tube (23) is provided in the outer tube (21), the insulating middle tube (23) extends from the proximal end to the distal end, a conductive part (13) is provided in the insulating middle tube (23), the conductive part (13) extends from the proximal end to the distal end, a terminal of the proximal end of the conductive part (13) can be connected to an external power source; a boosting tube (22) is provided between the proximal end of the outer tube (21) and the insulating middle tube (23), the distal end of the boosting tube (22) and the proximal end of the insulating middle tube (23) are connected with each other; the distal end of the conductive part (13) is provided with an insulating part (12), a conductive head (11) is distributed on the insulating part (12), and the conductive head (11) is connected with the conductive part (13) to achieve a conductive function, and the conductive part (13) also has a function of supporting a stent; when the stent is compressed, it is located in a space between the distal end of the conductive part (13) and the outer tube (21), the front handle (30) is connected to the proximal end of outer tube (21), and is moved backwards along the boosting tube (22), to drive the outer tube (21) to move backwards to release the stent;
the conductive part (13) is a hollow tubular conductive part, or a conductive wire; and
the boosting tube (22) extends towards the proximal end and is connected with the
rear handle (31), and a positioning part (27) is provided between the front handle (30) and the rear handle (31).

2. The thermopuncture stent implantation device according to claim 1, wherein there is a gap between the insulating part (12) and the conductive part (13), the conductive head (11) is provided at a terminal of the distal end of the implantation device, one end of the conductive head (11) extends from the distal end to the proximal end to enter the gap between the insulating part (12) and the conductive part (13), and thus is connected with the conductive part (13) to achieve the conductive function, the other end of the conductive head (11) is covered on an outer surface of the insulating part (12).

3. The thermopuncture stent implantation device according to claim 1, wherein the terminal of the proximal end of the conductive part (13) is connected with a Luer connector (34) to implement liquid injection.

4. The thermopuncture stent implantation device according to claim 1, wherein the conductive part (13) is a nickel-titanium wire.

5. The thermopuncture stent implantation device according to claim 1, wherein the conductive part (13) is a metal material.

6. The thermopuncture stent implantation device according to claim 5, wherein the conductive part (13) is a stainless steel material.

7. The thermopuncture stent implantation device according to claim 1, wherein the outer tube (21) comprises a proximal outer tube (211) and a distal outer tube (212), the proximal outer tube (211) and the distal outer tube (212) are connected in a taper.

8. The thermopuncture stent implantation device according to claim 1, wherein an outer surface of the conductive part (13) at a distance from the conductive head (11) is covered with a resistance part (28).

9. The thermopuncture stent implantation device according to claim 1, wherein the conductive head (11) comprises two or four conductive wires, and the two or four conductive wires are evenly distributed within grooves on an outer surface of the insulating part (12).

10. The thermopuncture stent implantation device according to claim 1, wherein the other end of the conductive head (11) close to an outer side is completely covered on an outer surface of the insulating part (12), and when cutting with the conductive head (11), a cut surface of a wound is a circular surface.

11. The thermopuncture stent implantation device according to claim 1, wherein an outer surface of the conductive part (13) is covered with a riveting tube (29), one end of the conductive head (11) extends from the distal end to the proximal end to enter a gap between the insulating part (12) and the conductive part (13), and is connected with the conductive part (13) through the riveting tube (29) to implement a conductive function.

12. The thermopuncture stent implantation device according to claim 1, wherein a material of the insulating part (12) is ceramic.

## Patentansprüche

1. Thermopunktions-Stentimplantationsvorrichtung, wobei die Thermopunktions-Stentimplantationsvorrichtung ein proximales Ende und ein distales Ende, einen vorderen Griff (30) und einen hinteren Griff (31) aufweist, wobei das distale Ende des vorderen Griffs (30) mit einer äußeren Röhre (21) versehen ist, wobei sich die äußere Röhre (21) von dem proximalen Ende zu dem distalen Ende erstreckt, wobei ein Außendurchmesser des distalen Endes der äußeren Röhre (21) kleiner oder gleich 3,15 mm ist, wobei eine isolierende mittlere Röhre (23) in der äußeren Röhre (21) bereitgestellt ist, wobei sich die isolierende mittlere Röhre (23) von dem proximalen Ende zu dem distalen Ende erstreckt, wobei ein leitender Teil (13) in der isolierenden mittleren Röhre (23) bereitgestellt ist, wobei sich der leitende Teil (13) von dem proximalen Ende zu dem distalen Ende erstreckt, wobei ein Anschluss des proximalen Endes des leitenden Teils (13) mit einer externen Leistungsquelle verbunden werden kann; wobei eine Verstärkungsröhre (22) zwischen dem proximalen Ende der äußeren Röhre (21) und der isolierenden mittleren Röhre (23) bereitgestellt ist, wobei das distale Ende der Verstärkungsröhre (22) und das proximale Ende der isolierenden mittleren Röhre (23) miteinander verbunden sind; wobei das distale Ende des leitenden Teils (13) mit einem isolierenden Teil (12) versehen ist, wobei ein leitender Kopf (11) auf dem isolierenden Teil (12) verteilt ist und der leitende Kopf (11) mit dem leitenden Teil (13) verbunden ist, um eine leitende Funktion zu erfüllen, und der leitende Teil (13) außerdem eine Funktion zum Halten eines Stents aufweist; wobei, wenn der Stent zusammengedrückt ist, er sich in einem Zwischenraum zwischen dem distalen Ende des leitenden Teils (13) und der äußeren Röhre (21) befindet, wobei der vordere Griff (30) mit dem proximalen Ende der äußeren Röhre (21) verbunden ist und entlang der Verstärkungsröhre (22) nach hinten bewegt wird, um die äußere Röhre (21) anzutreiben, um sich nach hinten zu bewegen, um den Stent freizugeben;
wobei der leitende Teil (13) ein hohler röhrenförmiger leitender Teil oder ein leitender Draht ist; und
wobei sich die Verstärkungsröhre (22) in Richtung des proximalen Endes erstreckt und mit dem hinteren Griff (31) verbunden ist und ein Positionierungsteil (27) zwischen dem vorderen Griff (30) und dem hinteren Griff (31) bereitgestellt ist.

2. Thermopunktions-Stentimplantationsvorrichtung nach Anspruch 1, wobei ein Spalt zwischen dem isolierenden Teil (12) und dem leitenden Teil (13) vorhanden ist, wobei der leitende Kopf (11) an einem Anschluss des distalen Endes der Implantationsvorrichtung bereitgestellt ist, wobei sich ein Ende des leitenden Kopfs (11) von dem distalen Ende zu dem proximalen Ende erstreckt, um in den Spalt zwischen dem isolierenden Teil (12) und dem leitenden Teil (13) einzudringen, und somit mit dem leitenden Kopf (13) verbunden ist, um die leitende Funktion zu erfüllen, wobei das andere Ende des leitenden Kopfs (11) auf einer Außenoberfläche des isolierenden Teils (12) abgedeckt ist.

3. Thermopunktions-Stentimplantationsvorrichtung nach Anspruch 1, wobei der Anschluss des proximalen Endes des leitenden Teils (13) mit einem Luer-Verbinder (34) verbunden ist, um das Einspritzen von Flüssigkeit zu implementieren.

4. Thermopunktions-Stentimplantationsvorrichtung nach Anspruch 1, wobei der leitende Teil (13) ein Nickel-Titan-Draht ist.

5. Thermopunktions-Stentimplantationsvorrichtung nach Anspruch 1, wobei der leitende Teil (13) ein Metallmaterial ist.

6. Thermopunktions-Stentimplantationsvorrichtung nach Anspruch 5, wobei der leitende Teil (13) ein Edelstahlmaterial ist.

7. Thermopunktions-Stentimplantationsvorrichtung nach Anspruch 1, wobei die äußere Röhre (21) eine proximale äußere Röhre (211) und eine distale äußere Röhre (212) umfasst, wobei die proximale äußere Röhre (211) und die distale äußere Röhre (212) in einem Konus verbunden sind.

8. Thermopunktions-Stentimplantationsvorrichtung nach Anspruch 1, wobei eine Außenoberfläche des leitenden Teils (13) in einem Abstand von dem leitenden Kopf (11) mit einem Widerstandsteil (28) abgedeckt ist.

9. Thermopunktions-Stentimplantationsvorrichtung nach Anspruch 1, wobei der leitende Kopf (11) zwei oder vier leitende Drähte umfasst und die zwei oder vier leitenden Drähte innerhalb von Nuten auf einer Außenoberfläche des isolierenden Teils (12) gleichmäßig verteilt sind.

10. Thermopunktions-Stentimplantationsvorrichtung nach Anspruch 1, wobei das andere Ende des leitenden Kopfs (11) nahe einer Außenseite auf einer Außenoberfläche des isolierenden Teils (12) vollständig abgedeckt ist und wobei, wenn mit dem leitenden Kopf (11) geschnitten wird, eine Schnittfläche einer Wunde eine kreisförmige Fläche ist.

11. Thermopunktions-Stentimplantationsvorrichtung nach Anspruch 1, wobei eine Außenoberfläche des leitenden Teils (13) mit einer Nietröhre (29) abgedeckt ist, wobei sich ein Ende des leitenden Kopfs (11) von dem distalen Ende zu dem proximalen Ende erstreckt, um in einen Spalt zwischen dem isolierenden Teil (12) und dem leitenden Teil (13) einzudringen, und über die Nietröhre (29) mit dem leitenden Teil (13) verbunden ist, um eine leitende Funktion zu implementieren.

12. Thermopunktions-Stentimplantationsvorrichtung nach Anspruch 1, wobei ein Material des isolierenden Teils (12) Keramik ist.

## Revendications

1. Dispositif d'implantation de stent par thermoponction, dans lequel le dispositif d'implantation de stent par thermoponction présente une extrémité proximale et une extrémité distale, une poignée avant (30), une poignée arrière (31), l'extrémité distale de la poignée avant (30) est pourvue d'un tube externe (21), le tube externe (21) s'étend de l'extrémité proximale à l'extrémité distale, un diamètre externe de l'extrémité distale du tube externe (21) est inférieur ou égal à 3,15 mm, un tube médian isolant (23) est fourni dans le tube externe (21), le tube médian isolant (23) s'étend de l'extrémité proximale à l'extrémité distale, une partie conductrice (13) est fournie dans le tube médian isolant (23), la partie conductrice (13) s'étend de l'extrémité proximale à l'extrémité distale, une borne de l'extrémité proximale de la partie conductrice (13) peut être connectée à une source de puissance externe ; un tube d'amplification (22) est fourni entre l'extrémité proximale du tube externe (21) et le tube médian isolant (23), l'extrémité distale du tube d'amplification (22) et l'extrémité proximale du tube médian isolant (23) sont raccordées l'une à l'autre ; l'extrémité distale de la partie conductrice (13) est pourvue d'une partie isolante (12), une tête conductrice (11) est distribuée sur la partie isolante (12), et la tête conductrice (11) est connectée avec la partie conductrice (13) pour obtenir une fonction conductrice, et la partie conductrice (13) a également une fonction de support d'un stent ; lorsque le stent est compressé, il est situé dans un espace entre l'extrémité distale de la partie conductrice (13) et le tube externe (21), la poignée avant (30) est raccordée à l'extrémité proximale du tube externe (21), et est déplacée vers l'arrière le long du tube d'amplification (22), pour entraîner le tube externe (21) pour qu'il se déplace vers l'arrière pour libérer le stent ;
la partie conductrice (13) est une partie conductrice tubulaire creuse, ou un fil conducteur ; et
le tube d'amplification (22) s'étend vers l'extrémité proximale et est raccordé à la poignée arrière (31), et une partie de positionnement (27) est fournie entre la poignée avant (30) et la poignée arrière (31).

2. Dispositif d'implantation de stent par thermoponction selon la revendication 1, dans lequel il n'y a pas d'espace entre la partie isolante (12) et la partie conductrice (13), la tête conductrice (11) est fournie au niveau d'une borne de l'extrémité distale du dispositif d'implantation, une extrémité de la tête conductrice (11) s'étend de l'extrémité distale à l'extrémité proximale pour entrer dans l'espace entre la partie isolante (12) et la partie conductrice (13), et est ainsi connectée à la partie conductrice (13) pour obtenir la fonction conductrice, l'autre extrémité de la tête conductrice (11) est couverte sur une surface externe de la partie isolante (12).

3. Dispositif d'implantation de stent par thermoponction selon la revendication 1, dans lequel la borne de l'extrémité proximale de la partie conductrice (13) est connectée à un raccord Luer (34) pour effectuer une injection de liquide.

4. Dispositif d'implantation de stent par thermoponction selon la revendication 1, dans lequel la partie conductrice (13) est un fil en nickel-titane.

5. Dispositif d'implantation de stent par thermoponction selon la revendication 1, dans lequel la partie conductrice (13) est un matériau en métal.

6. Dispositif d'implantation de stent par thermoponction selon la revendication 5, dans lequel la partie conductrice (13) est un matériau en acier inoxydable.

7. Dispositif d'implantation de stent par thermoponction selon la revendication 1, dans lequel le tube externe (21) comprend un tube externe proximal (211) et un tube externe distal (212), le tube externe proximal (211) et le tube externe distal (212) sont raccordés en cône.

8. Dispositif d'implantation de stent par thermoponction selon la revendication 1, dans lequel une surface externe de la partie conductrice (13) à une distance de la tête conductrice (11) est couverte avec une partie de résistance (28).

9. Dispositif d'implantation de stent par thermoponction selon la revendication 1, dans lequel la tête conductrice (11) comprend deux ou quatre fils conducteurs, et les deux ou quatre fils conducteurs sont équitablement répartis au sein de rainures sur une surface externe de la partie isolante (12).

10. Dispositif d'implantation de stent par thermoponction selon la revendication 1, dans lequel l'autre extrémité de la tête conductrice (11) proche d'un côté externe est complètement couverte sur une surface externe de la partie isolante (12), et lors d'une découpe à l'aide de la tête conductrice (11), une surface de découpe d'une blessure est une surface circulaire.

11. Dispositif d'implantation de stent par thermoponction selon la revendication 1, dans lequel une surface externe de la partie conductrice (13) est couverte avec un tube à river (29), une extrémité de la tête conductrice (11) s'étend à partir de l'extrémité distale jusqu'à l'extrémité proximale pour entrer dans un espace entre la partie isolante (12) et la partie conductrice (13), et est connectée à la partie conductrice (13) par le biais du tube à river (29) pour effectuer une fonction conductrice.

12. Dispositif d'implantation de stent par thermoponction selon la revendication 1, dans lequel un matériau de la partie isolante (12) est en céramique.
